# EUROPEAN PATENT SPECIFICATION

(11) **EP 2 229 757 B1**
(45) Date of publication and mention of the grant of the patent: **14.12.2016**
(21) Application number: 07866787.0
(22) Date of filing: 06.12.2007
(51) Int. Cl.: A61B 5/00, H04B 1/7163, H04W 84/18, H04L 29/08

(54) **REPROGRAMMABLE WIRELESS SENSOR SYSTEM FOR VITAL SIGNS MONITORING**
UMPROGRAMMIERBARES DRAHTLOSES SENSORSYSTEM FÜR DIE ÜBERWACHUNG VON LEBENSZEICHEN
CAPTEUR SANS FIL REPROGRAMMABLE POUR LA SURVEILLANCE DES SIGNES VITAUX

(30) Priority: 28.11.2007 IT PI20070134
(43) Date of publication of application: 22.09.2010
(73) Proprietor: Cozzani, Roberto, 56028 San Miniato (PI) (IT); Mucchi, Lorenzo, 50053 Empoli (FI) (IT)
(72) Inventor: Cozzani, Roberto, 56028 San Miniato (PI) (IT); Mucchi, Lorenzo, 50053 Empoli (FI) (IT)
(74) Representative: Emmi, Mario
(86) International application number: PCT/IT2007/000855
(87) International publication number: WO 2009/069163

(56) References cited:
- US-A1- 2005 090 200
- US-A1- 2006 058 017
- US-A1- 2007 254 728
- D'SOUZA M ET AL: "Architecture of wireless sensor node using novel ultra-wideband modulation scheme" DIGITAL SYSTEM DESIGN, 2004. DSD 2004. EUROMICRO SYMPOSIUM ON RENNES, FRANCE AUG. 31 - SEPT. 3, 2004, PISCATAWAY, NJ, USA,IEEE, 31 August 2004 (2004-08-31), pages 579-586, XP010723548 ISBN: 978-0-7695-2203-6
- GIANNAKIS G B ET AL: "Ultra-wideband communications - An idea whose time has come" IEEE SIGNAL PROCESSING MAGAZINE, IEEE SERVICE CENTER, PISCATAWAY, NJ, US, vol. 21, no. 6, 1 November 2004 (2004-11-01), pages 26-54, XP011122123 ISSN: 1053-5888
- NAN GUO ET AL: "A UWB Radio Testbed-System Design and Implementation" SYSTEM THEORY, 2006 PROCEEDING OF THE THRITY-EIGHTH SOUTHEASTERN SYMPO SIUM ON MARCH 5, 2006, PISCATAWAY, NJ, USA,IEEE, 5 March 2006 (2006-03-05), pages 299-303, XP010909517 ISBN: 978-0-7803-9457-5

## Description

### Field of invention

The invention deals with the technical sector related to the continuous monitoring systems of the vital signs of the patients by using wireless devices. In particular, we refer to a wireless network for measuring and transmitting human bio-potential vital signs through base band electromagnetic signals. Each sensor is coupled to normal electrode patch and connected via radio signal to a base unit that receives and processes the vital signs coming from more than one sensor and sends them to a conventional monitor for the displaying of the data.

### State of the art

The electronic instruments for the measurement and the monitoring of the vital signs (LPs) of the patients are commonly used in a hospital ward. The ECG monitors allow the sanitary operators to control the vital parameters of the patients (cardiac frequency, oxygen saturation, invasive and non invasive blood pressure, breath frequency, ECG trace with 3 or 5 leads, etc.). In particular, each patient is connected to an ECG monitor in the intensive care ward in order to allow a strict surveillance by the sanitary operators and an immediate intervention when needed. Each ECG monitor is equipped with several cables that must be connected to the patient in order to measure the vital signs. The standard typology can be described as follows:
- Cables (up to 10) for the ECG trace, connected to the chest of the patient through specific patches equipped with an electrode inside;
- Cable for the oxygen saturation measurement, connected to one of the patient's fingers;
- Cable for the non invasive blood pressure measurement, connected to the arm of the patient;
- Cable for the invasive blood pressure measurement, connected to an artery catheter.

It is easy to note that such high number of cables strongly limits the mobilit of the patient hampering both his voluntary and involuntary movements. Consequently, the sanitary operators are continuously forced to control and to reposition the cables. Moreover, the cables need continuous cleaning and maintenance. The telemetry systems for the wireless ECG monitoring are already present. However, these systems are not cost efficient and their implementation shows a high degree of complexity. Moreover, they do not take into account the interferences coming from other wireless devices or systems. Therefore these systems are not optimized for a continuous monitoring of the patients. Some of them are not completely mobile (wireless), because the electrodes are not composed by a transceiver, but they are wired to a transmitter that has to be carried by the patient itself.

A programmable electrodes wireless system for the bio-electric parameters monitoring has been proposed in a previous patent US pat. No. 2006/0058017A1 (by Mohammad et al.). Such patent deals with a programmable wireless system for bio-electric signals acquisition (ECG). That system is thought for the one-shot acquisition of the bio-electric signals of the patient, but it is not suitable for a continuous measurement of those parameters.

The system proposed by Mohammad et al. includes a base unit connected to a standard monitor and to a plurality of remote programmable transceivers, plugged to a patch electrodes. The wireless transceiver sends the bio-potential signals to the base unit for the displaying. The signals coming from different transceivers are sent through a common frequency carrier, but in a different time slot. A different frequency carrier can be selected in order to display the ECG of another patient at the same time. The presence of a frequency carrier makes the radio frequency (RF) stadium mandatory, both in the sensor and in the base unit. The presence of the RF box implies very complex devices, higher power consumption and the necessity of an acquisition and tracking box.

### Summary of the invention

It is an object of the proposed invention to provide a method, according to claim 1, and a device, according to claim 14, for permanent monitoring vital signs of a patient.

### Description of the invention

The object of the proposed invention is a network of reprogrammable wireless devices to be used for continuously monitoring the vital signs of one or more patients, each one coupled with conventional patch electrodes and connected to a base unit (BU). The base unit receives and processes the vital signs coming from the sensors and sends them to a conventional monitor for the data displaying. A base band impulse signal is used for the transmission of the information. The proposed base band communication technique offers features that are not present in previous patents. The goal of the present invention is not the substitution of the existent telemetry systems, but the realization of a continuous monitoring of the vital signs by using a more flexible, cheap and convenient system, in particular for patients that cannot leave the bed or have very limited mobility in a intensive therapy ward or similar sanitary structure.

The baseband impulsive system has the following advantages:
- No carrier is needed for the transmission; this assures a less complex and less expensive device;
- The ultra wideband radio transmissions do not normally suffer from the interferences due to other wireless systems (medical and non medical, such as Wi-Fi, UMTS, GSM, everything which is present in the ISM band, etc.);
- The proposed system does not imply the use of licensed/reserved frequency bands (very expensive) for a correct and secure work (the system is *license free*);
   ∘ The system proposed by Mohammad et al. implies the exclusive reservation (paying) of a portion of frequency spectrum for the correct (no interference) work, or, alternatively, it implies the use of the system in the ISM band (free band), but in this case it is subject to interference from other wireless telecommunication systems (this is very dangerous because it can threaten the transmission of the vital signs of the patients and put their lives at risk);
- The number of contemporary active sensors on several patients can be programmed; this implies more efficiency and flexibility when a simultaneous monitoring over more patients in the same room is needed;
- The amplitude of the operation range of the wireless sensor network can be programmed; this implies an improvement in the range of the potential applications;
- Low battery consumption which makes the system particularly suitable for the continuous monitoring over the time of the vital signs;
- The use of baseband impulsive signals allows the localization of each sensor with few centimeters approximation;
- The sensors are always reprogrammable; for example, each sensor can be reprogrammed in order to occupy different positions on the chest of the patient; this implies a better flexibility of the system and decreases significantly the interruptions of monitoring for the substitution of the electrodes during the work;
- More than one base unit can coexist at the same time without any conflict or interference.

The proposed invention is particularly suitable for the permanent (wireless) monitoring of the bio-electrical signals of the patients in a hospital ward or similar structure. For example, in a heart intensive therapy hospital ward where the patients are forced to stay in the bed and to be continuously ECG monitored because of their critical health conditions. The proposed solution aims to minimize the stress felt by the patient and caused by the numerous cables which connect his/her body to the monitor and give him/her the feeling of being "more sick.". At the same time, it aims to reduce the time that the sanitary operators employ for repositioning and cleaning of the cables. The time saved can be employed more efficiently in other activities. In the modern concept of hospital the patient must be provided with a more comfortable and less traumatic staying, so that he can be motivated and helped to recover as soon as possible. The cost efficiency of this system is easy to see: the fact that a patient recovers more quickly and spends fewer days in the hospital allows the public sanitary structure to save a significant amount of money.

The main innovative aspect of the invention is the sensor network used for the continuous monitoring of the vital signs of the patients; such sensors transmit the information by means of radio impulsive signal (ultra wideband).

The system is composed by a plurality of individual wireless reprogrammable sensors, each one positioned on or inside conventional medical auxiliaries that allow conduction of the electrical signal from the body to the sensor (patch electrodes, needles for electromyography, metallic caps for EEG, etc.). "Medical auxiliary" refers here to the medical object which is normally positioned on the body of the patient and is used for the monitoring of the vital signs (ECG, EEG, electromyography, etc.). The system is also composed by a base unit that includes a wireless transceiver used for the transmission and the reception of the baseband signals from the sensors.

The base unit transmits a global time signal to all the sensors. The global time signal is needed for the synchronization between the base unit and all the individual sensors. Once the synchronization is reached, the base unit and all the individual sensors share the same time intervals (begin and end of each time slot).

The baseband messages sent from the base unit to the sensors include all the setting parameters, such as the time-hopping code and other parameters for the correct set up of the communication (transmission power level, number of impulses per bit, global time signal, etc.). The base unit and the sensors can communicate each other the control parameters by using a control channel time-divided from the data channel.

The base unit, during the configuration procedure, sends an unique time-hopping code to each sensor (the specific time-slots where the specific sensor is allowed to transmit), so that no interference is possible among the individual sensors. This time division multiplexing allows each sensor to transmit its data to the base unit by means of specific time slots, sharing the same baseband channel.

The base unit is provided with an interface that allows the connection to an ECG monitor for the displaying of the bio-electric signals of the patient. The interface can be connected to a conventional ECG monitor or it can be implemented inside a monitor. If a standard ECG monitor is used, the base unit is provided with an interface that formats the data coming from the sensors in order to let them be readable to the monitor. The standard monitor does not detect any difference between the signals coming from the standard wired cables and from the proposed wireless sensors.

The sensors are designed to be connected to patch electrodes. The patch electrodes are put on particular locations on the surface of the body of the patient. During the programming procedure, the sensors are installed one by one by the sanitary operator. When the sensor is switched on, it transmits a start-up impulse or impulses on the control channel. The base unit receives the signal and asks the operator (through a diplay) which is the location of that specific sensor on the body surface of the patient (following the normal procedure for the standard ECG exam).

The base unit is able to scan the time axis in order to acquire all the time locations (time slots) occupied by other active sensors owned by another base unit. Hence, the base unit assigns a specific time-hopping code to the specific sensor, which ends the transmission on the control channel and starts the information transmission over the data channel by using the assigned specific time slots (the data channel and the control channel are time divided). If another sensor (or several others sensors) must be installed, the operator can switch it (them) on following the same configuration procedure until the exact number of sensors for the specific exam of the patient is reached. Once all necessary sensors are installed and configured, the operator communicates to the base unit the command for ending the sensors configuration procedure. Now the monitoring of the vital signs can start.

The base unit and the sensors communicate each other by means of radio impulse base band signals. A single impulse is very short in time and it has a power spectral density according to the FCC specifications. More than one impulse can be used to represent an information bit, so that the total transmitted energy and consequently the operability range can be modified. Even the power level of a single pulse can be modified to obtain the same goal.

The electronic device that composes the wireless sensor includes a low noise amplifier, an analog/digital converter, a microcontroller, a digital signal processor (DSP), a memory, a buffer, an encoder/decoder, a battery supply, a baseband transceiver and an antenna. The wireless transceiver is composed by a correlator (including a template of the transmitted pulse), a local oscillator, a programmable delay generator, a modulator, a code generator and a pulse generator.

The base unit includes an antenna, a base band transceiver, an encoder/decoder, a micro controller coupled with a DSP (connected to the user interface, the I/O interface and the memory), a demultiplexer, a bank of buffers, a bank of digital/analog converters (one per sensor), a battery supply and an interface to the standard ECG monitor.

The transmitter includes a pulse generator, a programmable delay generator, a local oscillator, a code generator and a modulator. The data are sent to the modulator that creates the pulses train according to the time-hopping code sent to the sensor by the base unit during the configuration phase. The data are modulated by the baseband impulses train as specified by the time-hopping code of the specified sensor. The modulated signal is D/A converted, amplified and fed to the antenna for the transmission.

The receiver, inside the base unit, includes a correlator, composed by a multiplier, an integrator and a threshold revealer, a baseband signals processing platform composed by a DSP, a microcontroller, a memory, an acquisition and tracking control unit, a pulse generator, a programmable delay generator and a local oscillator. The signal coming from the sensor is first A/D converted and then correlated with a baseband pulses train template, according to the specified time-hopping code of the sensor. The output signal is then fed to the baseband processor that extracts and processes the data. The information data are then sent to the monitor for the displaying. The base unit stores in the memory the time-hopping code and the location of the each individual transmitting sensor.

The proposed wireless sensors system does not need a centralized coordination, unless it is specifically required by the end user of the invention. Each base unit can be used individually, no matter if other base units are already activated. The proposed invention has got a specific procedure in order to avoid the collisions between different base units. Moreover, the invention provides a specific procedure in order to avoid errors from the operator during the programming of the sensors.

A possible alternative solution is the use of a "super base unit" that manages more than one monitor (and thus more than one patient) at the same time. In such a case the properties of precise localization offered by the ultra wideband communications are extremely useful in order to help the base unit to reveal with higher precision when a particular sensor is owned by a particular patient. The patch electrodes, to which the sensors are connected, transmit continuously the bio-electric signals of the patient. It is important to protect the sensor against possible voltage shocks (for example during a defibrillation), that could damage the sensor.

The invention deals with a system for the permanent monitoring of the vital signs. Such system includes:
a. One or more wireless sensors for the monitoring of the vital signs; each sensor is equipped with a transceiver for transmitting and receiving baseband impulsive signals;
b. At least one base unit (BU) equipped with a transceiver for transmitting and receiving baseband impulsive signals to one or more of the mentioned sensors and connected to at least one monitor.

The system provides that at least one sensor and one base unit communicate each other by using baseband impulsive signals (ultra wideband signals) with a baseband multiplexing technique.

According to the invention a certain wireless sensor and a certain base unit communicate by means of impulsive broadband signals transmitted in assigned time slots, with a time or code division multiplexing communication. According to the invention a base unit transmits a global time signal to all the sensors in order to synchronize the time sequence of each sensor with the one generated by the base unit.

According to the invention the base unit, during the configuration phase, searches for a global time signal transmitted by another wireless devices of the same typology, and, if it is found, the base unit aligns its time sequence with the existing one so that they share the same time intervals.

According to the invention the base unit sends to each sensor managed by itself, the configuration parameters that guarantee the correct baseband communication by using a control channel time-divided or code-divided by the data channel. According to the invention the base unit assigns to each wireless sensor a specific code and/or a specific time slot where the sensor must transmit its impulses representing the vital signs revealed by the patient. That assignation occurs during a specific configuration procedure of the sensor which starts before the transmission of the information data of the bio-electric signals of the patient.

According to the invention the base unit provides an interface to a standard monitor which displays the bio-potential signals coming from the wireless sensors exactly in the same way it does when the signals come from conventional wired sensors. Alternatively a base unit can be connected to a dedicated monitor or it can be implemented directly inside a special monitor. According to the invention, a said wireless sensor is designed to be coupled with a path electrode that has to be put on specified locations on the surface of the body of the patient.

According to the invention, during the said configuration phase the operator locates the electrodes on the patient one by one. Each sensor, once switched on, transmits a start-up impulse or impulses in a said control channel. The base unit receives the above mentioned signal and asks the operator, through a display, for the exact position of the sensor on the body of the patient, and then it assigns a code to the sensor. When the code is acquired, the sensor ends to transmit in the control channel and starts to transmit the information data in the data channel by using the assigned code. The base unit has an archive for the storing of the codes and positions assigned to each sensor.

According to the invention the base unit is able to analyze all usable codes and/or time slots in order to acquire all the occupied codes or time slots by other sensors which are already active and not owned by the base unit. The base unit can now assign to a new sensor a free code or time slots where it can transmit without collisions.

According to the invention the base unit can repeat the configuration procedure described above until a free code or time slots are present or until the operator gives the end configuration procedure command.

According to the invention the same sensor can be configured more than more than once by a base unit and its setting parameters can be changed too.

According to the invention the configuration procedure can be started in every moment when needed by the operator, so that the operator can substitute or configure a new sensor without interrupting or altering the other active sensors which can continue to transmit the vital signs normally.

According to the invention a wireless sensor includes a low noise amplifier, an A/D converter, a microcontroller, a DSP, a memory, a buffer, an encoder/decoder, a battery for energy supply, a baseband transceiver and a transmitting/receiving antenna.

According to the invention a base unit includes a transmitting/receiving antenna, a baseband transceiver, an encoder/decoder, a microcontroller coupled with a DSP connected to a user interface, an I/O interface and a memory, a demultiplexer, a bank of buffers, a bank of D/A converters (one per sensor), a battery for energy supply and a monitor interface.

According to the invention a said wireless transceiver includes at least one programmable delay generator, a modulator, a code generator and a pulse generator.

According to the invention in a said wireless sensor, the data are sent to the modulator that creates the impulses train according to the time-hopping code assigned by the base unit to the sensor during the configuration phase. The data are modulated by several baseband radio impulses as specified by the time-hopping code assigned to the specific sensor. The modulated signal is D/A converted, amplified and fed to the antenna for the transmission.

According to the invention a receiver inside a base unit includes a correlator composed by a multiplier, an integrator and a threshold revealer, a baseband signal processing platform composed by a DSP, a memory, a microcontroller, an acquisition and tracking control unit, a pulse generator, a programmable delay generator and a local oscillator. The signal coming from a sensor is firstly A/D converted and correlated with a impulses train template according to the assigned time-hopping code. Then the signal is fed to the baseband processor that extracts and processes the information data which are then sent to the monitor for the display.

According to the invention, a said configuration procedure includes specific steps to avoid the interference from signals owned by other base units, so that more than one base unit can be used independently of the other already active base units in the same range of operability.

According to the invention a said sensor configuration procedure provides an alarm signal in the case that two sensors have the same position assigned from the operator.

According to the invention all or a part of the components of a wireless sensor are included in one or more integrated microprocessor in order to minimize the device dimensions.

According to the invention a base unit can use the particular characteristic of the baseband broadband signals to be able to locate a transmitter in order to identify with higher precision each sensor.

According to the invention a base unit is used to manage the signals of the sensors located on a single patient or alternatively a same base unit can include more than one modules so that it can manage the signals of the sensors positioned on more patients at the same time. In such case the property of the baseband impulsive signals of high resolution in locating the transmitter of the sensor is very helpful.

### Detailed description of an example of application

In the following example a possible application of the invention is described. In this example a time division multiplexer was used for an ECG continuous monitoring.

According to the annexed figures, the system includes three sensors 1.1-1.3 positioned on the body surface of a patient 1.6. The sensors 1.1-1.3 acquired the ECG signals from the body and transmits them to a base unit 1.4. The base unit is connected to a standard ECG monitor 1.5 for the display of the signals. The sensors 1.1-1.3 receive configuration commands from the base unit 1.4 by using a wireless transmission method. The sensors send the ECG signals to the base unit 1.4 by using the same transmission method. The system avoids the use of the bulky and sometimes dangerous cables.

The devices 1.1-1.3 of Fig. 1 are composed by a plurality of individual wireless remotely reprogrammable sensors 3.2. Each sensor is designed to be coupled with a conventional patch electrode 3.3 (for example a 3M red-dot) used for the standard ECG monitoring. The detailed scheme of a sensor is shown in Fig. 4. The base unit includes a wireless transceiver for transmitting and receiving signals from a plurality of individual sensors. The detailed scheme is shown in Fig. 6.

The wireless sensors receive a global time signal from the base unit for the synchronization purpose.

The wireless sensors and the base unit share the configuration procedure, the scanning procedure for revealing other active base units and the data transmission procedure.

The wireless sensors transmit the bio-electrical signals of the body to the base unit by means of baseband radio ultra wideband impulses.

The sensors are distinguished by using a time division multiplexing technique, i.e., each sensor has a different time-hopping code that is not overlapped in any part to the others. The sensors owned by a base unit are distinguished by different time-hopping codes. This fact makes it possible to use these ECG monitoring systems over more patients at the same time in the same range of operability.

The base unit receives the ECG signal from each electrode in a specific time interval (time slot). The ECG signal is demodulated, decoded (with error correction) and digitally processed. During this process all the desired parameters setting needed by the signal are applied (amplification, filtering etc.). The signal is than analog converted for the displaying on a standard ECG monitor.

The base unit has a specific interface to the standard ECG monitor in order to make the link between the sensors and the base unit to the standard ECG monitor completely transparent.

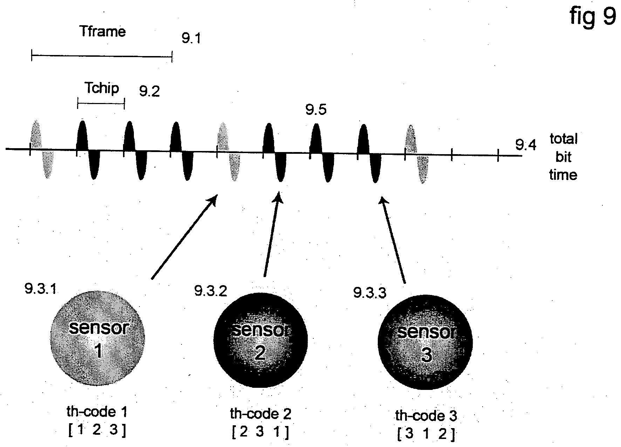

In the wireless communication between the base unit 1.4 and the sensors 3.2 according to the time-hopping technique used in a common ultra wideband carrierless channel, the exchanged data are sent by means of a baseband (time-narrow) impulses train. Each wireless sensor 3.2 transmits an ECG signal in specific time intervals according to the time-hopping code as stated in Fig. 9. The base unit 1.4 transmits the control commands to the sensors in a specific time interval called control channel 8.4.1, where all the sensors synchronize themselves. The time-hopping codes allocation and the other transmission control information are managed and controlled by the base unit 1.4.

Although the time division multiplexing (time-hopping) is the preferred technique for the division/separation of the sensors and the base units, other solutions can be used. An example of an alternative transmission technique is the well known direct sequence (DS) or the code division multiplexing (CDMA). The selection of the time-hopping technique is due to fact that the vital signs must be preserved as much as possible. The code division multiplexing does not avoid the collisions among the impulses of different sensors, although it makes them orthogonal. But, the orthogonal property can be lost because of synchronization errors or impairments of the radio channel.

The information transmitted by the base unit 1.4 includes also configuration commands for the wireless sensors 3.2. For example, these commands can provide or change the position of the sensor on the body of the patient, the transmission power level, the number of impulses per bit, the synchronization and timing of the signal, etc. Preferably, during the configuration procedure the base unit 1.4 sends a global timing signal for the synchronization of all the wireless sensors 3.2 owned by the base unit 1.4.

The detailed procedure of the design of the radio link for the messages exchange between the base unit 1.4 and the sensors 3.2 can be implemented in several ways inside the idea of the present invention.

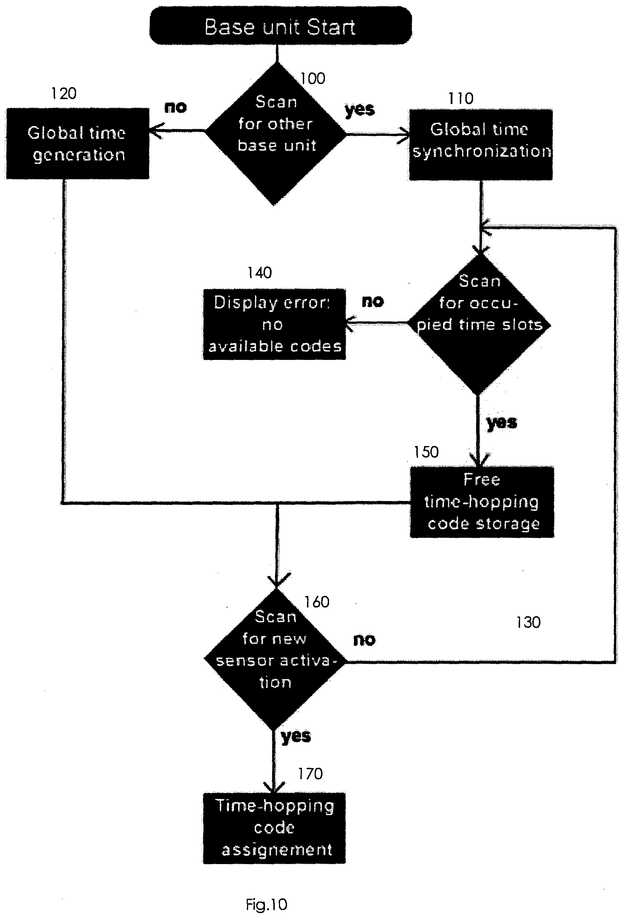

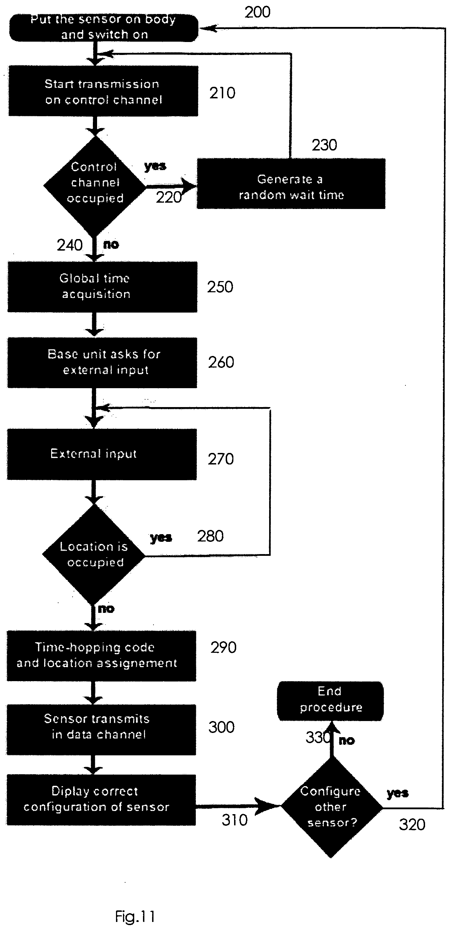

A possible configuration procedure for the base unit as well as the sensors is described in the following referring to the Figs. 10 and 11:
The base unit is plugged in the standard ECG monitor and switched on; the related sensors are off.

Firstly the base unit makes a scanning in order to reveal other active (transmitting) sensors owned by other base units (100).

If the base unit receives a signal from one or more sensors it implies that at least another base unit is present in the range of operability. The global timing of the already active base unit is acquired (110), so that all the base units share the same start and end time slots in the time axis.

If there is no other base unit in the range of operability the global timing is internally generated (120) and the procedure can go ahead with the next step (150).

If other transmitting sensors are revealed, the base unit makes a scanning in order to get all the occupied time slots locations (130);
The base unit memorizes a list of free time slots locations (150), in order to generate correctly its own time-hopping codes to be assigned to its sensors during the configuration procedure;
If all the time slots are occupied the base unit displays an alarm/error message (140). If one or more time slots are available, the procedure goes ahead with the next step (160).

The base unit continuously scans the control channel (160) in order to reveal configuration signals from the sensors; if a sensor starts to transmit in the control channel, its configuration procedure starts (170) in order to assign it the code.

In order to configure the sensors (Fig. 11) the sanitary operator locates a sensor on a specific position on the body of the patient and switches it on (200); the sensor starts to transmit on the control channel (210), i.e., it transmits a known signal with a locally generated timing. In fact, the sensor is not synchronized with the global timing of the base unit. The control channel could not be free. The base unit listens to the control channel and receives the signal of the sensor. If the control channel is already busy with another previous request (230), the base unit answers to the sensor with a WAIT signal; the sensor waits a random (230) time, and then tries again to transmit on the control channel;
If the control channel if free (240), the synchronization procedure starts and the global timing is acquired by the sensor (250);
Once the synchronization and timing procedure is ended, the base unit asks (displays) the operator for the location (typology) of the sensor on the body (260);
The operator digits the exact position (function) of the sensor through the I/O interface (270);
If the sensor position is already assigned, the base unit displays an error signal (280) and waits for another input in order to identify the sensor;
The base unit sends to the sensor its personal and unique time-hopping code, and then memorizes the code and the location of the sensor (290);
The sensor starts the data transmission (300);
The operator is informed by the base unit (through the user interface) that the sensor is correctly configured (310);
The operator switches on another sensor and repeats the procedure (200) until all the desired sensors (for the specific clinic exam) are configured.

When all the desired sensors are configured (320), the operator gives the "end configuration" signal to the base unit (330);
When the end configuration signal is received, the base unit does not accept other signals coming from other sensors;
The base unit starts to process the signals coming from its own sensors and sends them to the standard ECG monitor for the displaying;
This configuration/programming procedure of one or more sensors can be reopened if a sensor has a malfunction and must be substituted, or when other sensors must be added.

Each patch electrode 3.3 is coupled with its sensor 3.2.

The pin 3.4 conducts the electric impulses compared to the ground potential reference in order to measure the bio-potential difference between the signal in the pin and the ground reference.

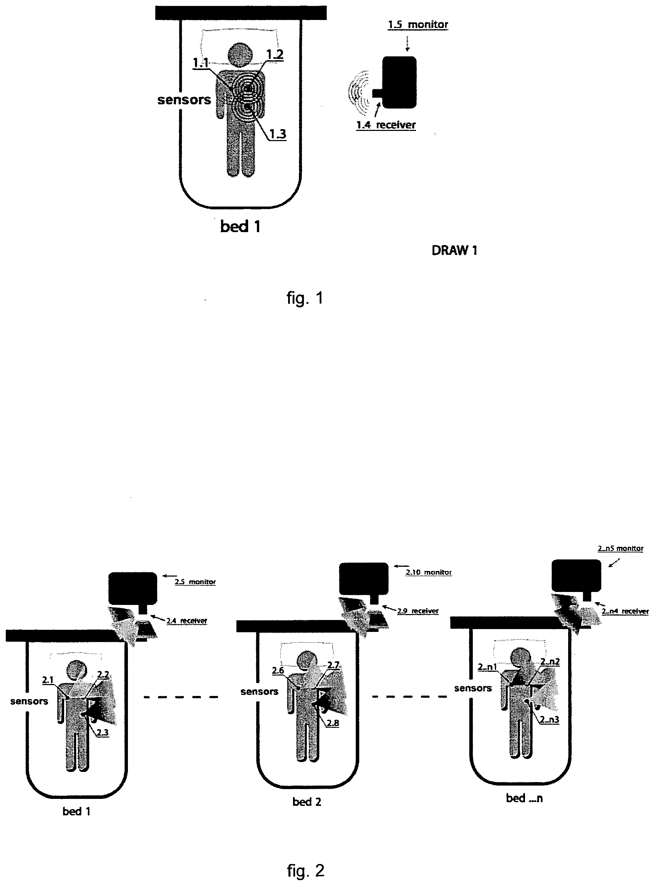

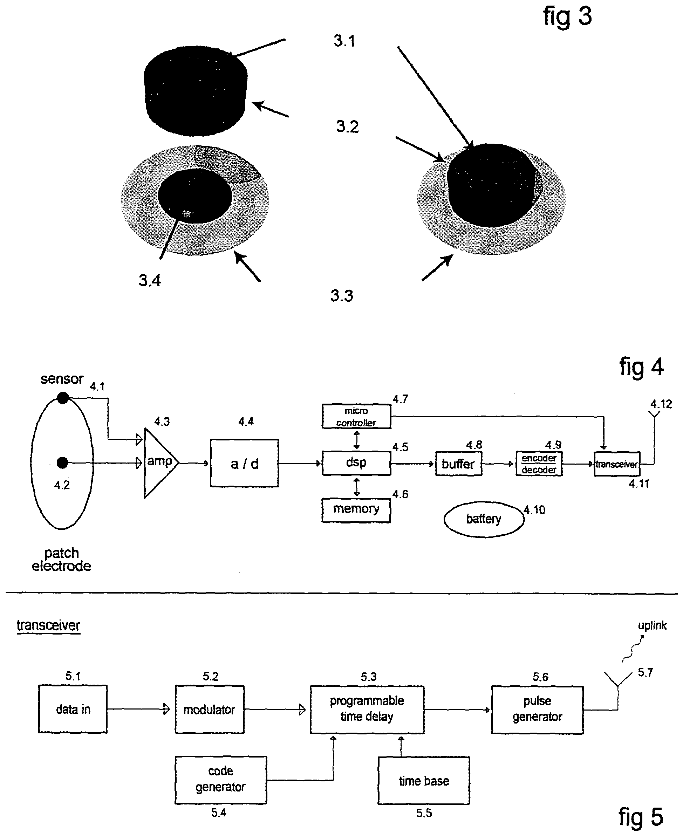

In Fig. 4 the wireless sensors (Figs. 1-3) block diagram is shown. The wireless sensor is plugged on the patch electrode 3.3. The electric signal taken by the electrode 3.4 is fed to a low noise amplifier 4.3. The analog signal is digitally converted by the A/D converter 4.4. The digital signal is fed to a baseband device/processor composed by a microcontroller 4.7, a DSP 4.5 and a memory 4.6. The microcontroller 4.7 processes also commands and messages coming from the base unit and executes the instructions stored in the memory 4.6. The memory also stores the time-hopping code assigned by the base unit during the configuration procedure. The processed ECG signal is fed to a buffer 4.8, then to the encoder/decoder 4.9 and finally to the baseband transceiver 4.11 for the transmission through an UWB low power antenna. A battery 4.10, preferably rechargeable, with the negative pole connected to the ground reference, provides all the components with the energy supply. The microcontroller/DSP 4.5/4.7 processes the assigned time-hopping code and controls the messages coming from the base unit. When the sensor 3.2 is switched on, the microcotroller/DSP 4.5/4.7 starts the transmission of the configuration signal on the control channel as provided by the said configuration procedure.

All or some of the components shown in Fig. 4 can be assembled in one or more microchips in order to minimize the dimension of the wireless sensor 3.2.

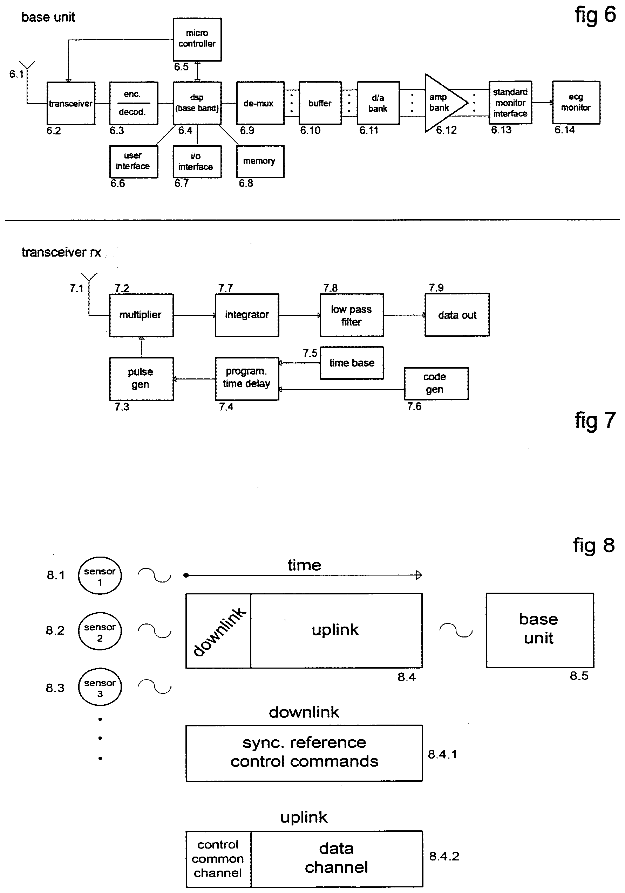

The transmitting section of the transceiver is shown in Fig. 5. The transmitter in Fig. 4 modulates 5.2 the input signal 5.1 and sends it to a programmable delay generator 5.3 which creates the time sequence according to the assigned time-hopping code. Then the signal is fed to a impulses generator 5.6 and sent to the antenna 5.7 for the transmission. A radio frequency stadium is not needed at all. The base unit scheme is presented in Fig. 6. The base unit 1.4 transmits commands to all the sensors 1.1-1.3 and lets each of those to transmit the own ECG information data individually (time-hopping code division multiple access). The base unit 1.4 receives the ECG signals from the sensors under its operation range. The received signal is demodulated, decoded, error corrected, demultiplexed, stored and analog converted in order to recover the data sent by the sensors and interface them to the standard ECG monitor 1.5. The base unit transmits also the time-hopping codes to the sensors, in order to allow a continuous transmission without collisions. The base unit can also send control commands to the sensors to change the transmission power level or the number of pulses per bit in order to modify the range of operability or improve the signal quality if needed.

The base unit 1.4 has a low power antenna 6.1 and a baseband transceiver 6.2.

The receiving section of the transceiver 6.2 is detailed in Fig. 7. It includes a cross-correlator, composed by a multiplier 7.2, a impulses generator 7.3, a programmable delays generator 7.4, a global time reference generator 7.5, a code generator 7.6, an integrator 7.7 and a low pass filter 7.8. Moreover, the base unit includes an encoder/decoder 6.3, a platform composed by a microcontroller 6.5, a DSP 6.4, a memory 6.8, a user interface 6.6 and a I/O interface 6.7.

The user interface 6.6 can be composed by a display that shows the information for the sensors programming as well as the alarm or error conditions, a key pad for the user inputs, an alarm unit for informing acoustically for the alarm conditions (battery low, failure in configuring a sensor, etc.) and led for visually indicating alarm or error conditions. The ECG signals, received by the wireless transceiver, are demultiplexed by the demultiplexer 6.9. The data are then analog converted by the D/A converter 6.11 and amplified by the bank of amplifiers 6.12. The data are then fed to the interface 6.13 to the standard ECG monitor 6.14. The interface 6.13 has to translate the ECG signals opportunely in order to render them compatible with the conventional ECG monitor input signals, as they came from the conventional wired cables. It is important to note that the base unit 1.4 can be also implemented directly in a ECG monitor.

A possible transmission scheme (time-hopping code division multiple access) between the wireless sensors 1.1-1.3 and the base unit 1.4 is reported in Fig. 8. The information is transported by means of baseband radio impulses (ultra wideband technique). All the sensors 8.1-8.3 receive the synchronization commands (the global timing signal, the increase/decrease power level command, etc.) from the base unit in a specific time interval 8.4 (downlink channel 8.4.1). The sensors 8.1-8.3 transmit the data and the messages on the uplink channel 8.4.2 that is time divided from the downlink channel. The uplink channel 8.4.2 is composed by a control channel and a data channel. The uplink control and data channels are time divided 8.4.2.

The transmission of the signals of the sensors 8.1-8.3 in the uplink channel 8.4.2 is detailed in Fig. 9. The transmission is based on the time-hopping technique. Each bit time interval 9.4 is divided into several frames 9.1. Each frame is divided into several chips 9.2. The first sensor 9.3.1, whose assigned time-hopping code is [1 2 3], transmits its impulses in the first chip of the first frame, in the second chip of the second frame and in the third chip of the third frame. Using the same strategy the other sensors 9.3.2 and 9.3.3 transmit their impulses 9.5 without any collision. The impulses owned by a specific sensor are sent in different time slots inside a bit time interval 9.4. The specific time slots where the impulses have to be transmitted are represented by the time-hopping code. The bit time interval can be divided into a specific number of frames 9.1 that indicates how many impulses are used by each sensor in a single bit interval. Each frame can be divided into a specific number of chip times 9.2 that indicates how many sensors can be activated at the same time without any interference. Just to make an example, if each sensor used 10 impulses per bit (this value would also set the range of operability once the transmission power level has been fixed), 400 time-hopping codes could be allocated inside the range of operability and thus 400 different sensors could be activated at the same time.

It is important to note although in the example of application described above the proposed invention is used for a permanent ECG monitoring, it could also be used in order to monitor other typologies of bio-potential signals coming from the human body (EEG signals, electromyography signals, etc.). The goal of this invention is determined as referred in the annexed claims.

### Brief description of the annexed drawings

Fig. 1 - reports an application of the invention for the ECG permanent monitoring of a patient: 3 wireless sensors transmit the ECG signal to the base unit connected to a standard ECG monitor.
Fig. 2 - reports a plurality of patients ECG monitored at the same time as depicted in Fig. 1; the Fig. 2 illustrates that more sensors on more patients can be contemporarily activated without collisions.
Fig. 3 - shows how the sensors are coupled to the conventional patch electrode.
Fig. 4 - shows the block diagram scheme of a wireless sensor.
Fig. 5 - details the scheme of the transmission section of the transceiver of a wireless sensor.
Fig. 6 - shows the block diagram scheme of a base unit.
Fig. 7 - details the scheme of the receiving section of the transceiver of the base unit.
Fig. 8 - shows the channels used by the base unit and the wireless sensors for the data and control signals communication. Moreover, the figure illustrates the time division nature of the communication system.
Fig. 9 - shows an example of time-hopping codes allocation for the wireless sensors. The impulses owned by different sensors are located in different chip intervals inside a bit.
Fig. 10 - shows the flow chart diagram of the configuration procedure of a base unit.
Fig. 11 - shows the flow chart diagram of the configuration procedure of the wireless sensors owned by a base unit.

## Claims

1. A method for the permanent monitoring of the vital signs, comprising the operation of:
a. Positioning of one or more wireless sensors (1.1, 1.2, 1. 3) on the body surface of a patient (1.6) for the vital signs monitoring; each sensor being equipped with a transceiver (4.11) for transmitting and receiving baseband impulsive signals;
b. Switching on of at least one base unit-BU (1.4) equipped with a transceiver for transmitting and receiving baseband impulsive signals from/to one or more said sensors and connected to at least one monitor (1.5);
wherein at least one sensor and one base unit communicate with each other by using ultra wideband impulsive signals transmitted in assigned time slots, with a time or code division multiplexing communication with a baseband multiple access technique and **characterized by** the fact that it is further provided a scanning phase wherein the base unit (1.4) scans all the codes and/or time slots usable by a new sensor in order to acquire all the occupied codes or time slots from other active sensors (2.1, 2.2, 2.3) eventually owned by another base unit (2.4), and to assign to said new sensor a free code and/or free time slots where it can transmit.

2. A method, as in the claim 1, **characterized by** the fact that a said wireless sensor (1.1, 1.2, 1. 3) and a said base unit (1.4) communicate by means of broadband impulsive signals transmitted in distinct time intervals assigned to each device with a time division or code division multiple access technique.

3. A method as in claim 1 or 2, **characterized by** the fact that one base unit (1.4) transmits a global time reference signal to all the sensors (1.1, 1.2, 1. 3) in order to synchronize the time sequence of each sensor with the one of the base unit.

4. A method, according to claim 1, **characterized by** the fact that a base unit (1.4) during the configuration phase searches for a global time reference signal generated by another base unit (2.4) and if it exists, the base unit aligns its time sequence with this one.

5. A method as in one or more of the previous claims **characterized by** the fact that a base unit (1.4) sends the configuration parameters; such parameters are sent through a control channel (160) to each sensor owned by the base unit; the control channel is time divided or code divided by the data channel where the transmission of the information is based on a baseband technique.

6. A method as in one or more of the previous claims **characterized by** the fact that the base unit (1.4) assigns to each wireless sensor (1.1, 1.2, 1. 3) a specific code and/or a specific time-hopping code according to which the sensor must transmit the pulses of the bio-potential signal taken from the patient's body (1.6); the base unit assigns the code during a specific configuration procedure of the sensor which starts before the beginning of the information of the measured vital signs of the patient.

7. A method as in one or more of the previous claims **characterized by** the fact that a base unit (1.4) has an interface to a standard monitor (1.5) which displays the bio-potential signals coming from the wireless sensors (1.1, 1.2, 1. 3) exactly as they came from conventional cables; alternatively, a base unit is connected to a dedicated monitor or it is implemented in a dedicated monitor.

8. A method as in one or more of the previous claims **characterized by** the fact that during a said configuration phase the operator locates the sensors (1.1, 1.2, 1. 3) one by one on the surface of the patient's body (1.6); each sensor, once switched on, transmits a start up signal on the said control channel (160). The base unit (1.4) receives the said start up signal and asks the operator through a display for the location of the sensor over the body of the patient; then the base unit assigns the code to the sensor; the sensor ends to transmit over the control channel and starts the transmission of the information data on the data channel according to the assigned code; the base unit stores the code and the location of each active sensor in a memory (4.6).

9. A method as in one or more of the previous claims **characterized by** the fact that a base unit repeats a said configuration procedure until a free code and/or time slot exists or until the operator digits an end configuration command.

10. A method as in one or more of the previous claims **characterized by** the fact that each sensor can be reconfigured several times by a base unit; each sensor can be also reprogrammed with a different function or location depending on the specific desired exam.

11. A method as in one or more of the previous claims **characterized by** the fact that a configuration procedure can be restarted every moment, so that the operator can substitute a sensor or configure a new sensor without interrupting or altering the transmission of the other sensors, which can continue to transmit the vital signs monitoring.

12. A method, as in one or more of the previous claims, **characterized by** the fact that in a said wireless sensor the data are sent to the modulator that creates the pulses train, according to the code assigned to the sensor by the base unit during the configuration phase. The data are modulated by several baseband radio pulses as specified by the code of the specific sensor. The modulated signal is analog converted, amplified and sent to the antenna (5.7) for the transmission.

13. A method, as in one or more of the previous claims, **characterized by** the fact that a said configuration procedure of a sensor provides an alarm signal when the same location is assigned to two sensors; in this way errors in the positioning of the sensors by the operator can be avoided.

14. A device for the permanent monitoring of the vital signs, composed by:
a. One or more wireless sensors (1.1, 1.2, 1. 3) applicable on the body surface of a patient (1.6) for the vital signs monitoring; each sensor equipped with a transceiver for transmitting and receiving baseband impulsive signals;
b. At least one base unit-BU (1.4) equipped with a transceiver for transmitting and receiving baseband impulsive signals from/to one or more said sensors and connected to at least one monitor (1.5),
wherein at least one sensor (1.1, 1.2, 1. 3) and one base unit (1.4) comprises means for communicate with each other by using ultra wideband impulsive signals transmitted in assigned time slots, with a time or code division multiplexing communication with a baseband multiple access technique and **characterized by** the fact that the base unit (1.4) is configured for scanning all the codes and/or time slots usable by a new sensor in order to acquire all the occupied codes or time slots from other active sensors (2.1, 2.2, 2.3) eventually owned by another base unit (2.4), and to assign to said new sensor a free code and/or free time slots where it can transmit.

15. A device, as per claim 14, **characterized by** the fact that a wireless sensor includes a low noise amplifier (4.3), a analog/digital converter (4.4), a microcontroller (4.7), a DSP (4.5), a memory (4.6), a buffer (4.8), an encoder/decoder (4.9), a battery supply (4.10), a baseband transceiver and an antenna and the base unit includes an antenna (6.1), a baseband transceiver (6.2), an encoder/decoder (6.3), a microcontroller (6.5) coupled with a DSP (6.4) connected to a user interface, an I/O interface (6.7) and a memory (6.8), a demultiplexer (6.9), a bank of buffers, a bank of analog/digital converters (each per sensor), a battery supply and a monitor interface.

## Patentansprüche

1. Verfahren zur permanenten Überwachung der Vitalzeichen, umfassend den Vorgang des:
a. Positionierens von einem oder mehreren drahtlosen Sensoren (1.1, 1.2, 1.3) auf der Körperoberfläche eines Patienten (1.6) für die Vitalzeichenüberwachung, wobei jeder Sensor mit einem Sende-Empfangsgerät (4.11) zum Senden und Empfangen von Basisband-Impulssignalen ausgestattet ist;
b. Einschaltens von mindestens einer Basiseinheit BU (1.4), die mit einem Sende-Empfangsgerät zum Senden und Empfangen von Basisband-Impulssignalen zu/von einem oder mehreren der Sensoren ausgestattet ist und mit mindestens einem Monitor (1.5) verbunden ist;
wobei mindestens ein Sensor und eine Basiseinheit durch die Nutzung von Ultrabreitband-Impulssignalen, die in zugewiesenen Zeitschlitzen übertragen werden, miteinander kommunizieren, mit einer Zeit- oder Codemultiplex-Kommunikation mit einer Basisband-Vielfachzugriffstechnik und **dadurch gekennzeichnet, dass** ferner eine Scanphase vorgesehen ist, bei der die Basiseinheit (1.4) alle durch einen neuen Sensor nutzbaren Codes und/oder Zeitschlitze scannt, um alle belegten Codes oder Zeitschlitze von anderen aktiven Sensoren (2.1, 2.2, 2.3) zu erfassen, die letztendlich einer anderen Basiseinheit (2.4) gehören, und um dem neuen Sensor einen freien Code und/oder freien Zeitschlitz zuzuweisen, in dem er übertragen kann.

2. Verfahren nach Anspruch 1, **dadurch gekennzeichnet, dass** der drahtlose Sensor (1.1, 1.2, 1.3) und die Basiseinheit (1.4) mittels Breitband-Impulssignalen kommunizieren, die in unterschiedlichen Zeitintervallen übertragen werden, die jeder Vorrichtung mit einer Zeitmultiplex- oder Codemultiplex-Vielfachzugriffstechnik zugewiesen werden.

3. Verfahren nach Anspruch 1 oder 2, **dadurch gekennzeichnet, dass** eine Basiseinheit (1.4) ein globales Zeitreferenzsignal an alle Sensoren (1.1, 1.2, 1.3) überträgt, um den Zeitablauf eines jeden Sensors mit der Basisstation zu synchronisieren.

4. Verfahren nach Anspruch 1, **dadurch gekennzeichnet, dass** eine Basiseinheit (1.4) während der Konfigurationsphase nach einem globalen Zeitreferenzsignal sucht, das durch eine andere Basiseinheit (2.4) generiert wird, und falls es vorhanden ist, die Basiseinheit ihren Zeitablauf mit diesem abgleicht.

5. Verfahren nach einem oder mehreren der vorhergehenden Ansprüche, **dadurch gekennzeichnet, dass** eine Basiseinheit (1.4) die Konfigurationsparameter sendet; solche Parameter werden durch einen Steuerkanal (160) zu jedem Sensor gesendet, der zu der Basiseinheit gehört; der Steuerkanal ist durch den Datenkanal zeitlich unterteilt oder mittels Code unterteilt, wo die Übertragung von Informationen auf einer Basisbandtechnik basiert.

6. Verfahren nach einem oder mehreren der vorhergehenden Ansprüche, **dadurch gekennzeichnet, dass** die Basiseinheit (1.4) jedem drahtlosen Sensor (1.1, 1.2, 1.3) einen bestimmten Code und/oder einen bestimmten Zeitsprungcode zuweist, gemäß welchem der Sensor die Impulse des vom Körper des Patienten aufgenommenen Biopotential-Signals (1.6) übertragen muss; die Basiseinheit weist den Code während eines bestimmten Konfigurationsverfahrens des Sensors zu, das vor dem Beginn der Informationen der gemessenen Vitalzeichen des Patienten startet.

7. Verfahren nach einem oder mehreren der vorhergehenden Ansprüche, **dadurch gekennzeichnet, dass** die Basiseinheit (1.4) eine Schnittstelle zu einem Standardmonitor (1.5) aufweist, der die von den drahtlosen Sensoren (1.1, 1.2, 1.3) kommenden Biopotential-Signale genau so anzeigt, wie wenn sie von herkömmlichen Kabeln kämen; alternativ ist eine Basiseinheit mit einem dedizierten Monitor verbunden oder in einem dedizierten Monitor implementiert.

8. Verfahren nach einem oder mehreren der vorhergehenden Ansprüche, **dadurch gekennzeichnet, dass** der Bediener während der Konfigurationsphase die Sensoren (1.1, 1.2, 1.3) nacheinander auf der Oberfläche des Körpers des Patienten (1.6) anordnet; sobald er eingeschaltet ist, überträgt jeder Sensor auf dem Steuerkanal (160) ein Startsignal. Die Basiseinheit (1.4) empfängt das Startsignal und fragt den Bediener durch eine Anzeige nach der Position des Sensors am Körper des Patienten; die Basiseinheit weist dann dem Sensor den Code zu; der Sensor beendet die Übertragung über den Steuerkanal und beginnt die Übertragung der Informationsdaten auf dem Datenkanal entsprechend dem zugewiesenen Code; die Basiseinheit speichert den Code und die Position jedes aktiven Sensors in einem Speicher (4.6).

9. Verfahren nach einem oder mehreren der vorhergehenden Ansprüche, **dadurch gekennzeichnet, dass** eine Basiseinheit ein Konfigurationsverfahren wiederholt, bis ein freier Code und/oder Zeitschlitz vorhanden ist oder bis der Bediener einen Befehl zum Beenden der Konfiguration eingibt.

10. Verfahren nach einem oder mehreren der vorhergehenden Ansprüche, **dadurch gekennzeichnet, dass** jeder Sensor mehrmals durch eine Basiseinheit neu konfiguriert werden kann; jeder Sensor kann auch mit einer anderen Funktion oder Position abhängig von der spezifischen gewünschten Untersuchung neu programmiert werden.

11. Verfahren nach einem oder mehreren der vorhergehenden Ansprüche, **dadurch gekennzeichnet, dass** ein Konfigurationsverfahren jederzeit neu gestartet werden kann, sodass der Bediener einen Sensor ersetzen oder einen neuen Sensor konfigurieren kann, ohne die Übertragung der anderen Sensoren zu unterbrechen oder zu verändern, die weiterhin die Überwachung der Vitalzeichen übertragen können.

12. Verfahren nach einem oder mehreren der vorhergehenden Ansprüche, **dadurch gekennzeichnet, dass** in einem drahtlosen Sensor die Daten an den Modulator gesendet werden, der die Impulsfolge erzeugt, gemäß dem Code, der dem Sensor während der Konfigurationsphase von der Basiseinheit zugewiesen wird. Die Daten werden durch mehrere Basisband-Funkimpulse wie durch den Code des jeweiligen Sensors spezifiziert moduliert. Das modulierte Signal wird analog umgewandelt, verstärkt und für die Übertragung an die Antenne (5.7) gesendet.

13. Verfahren nach einem oder mehreren der vorhergehenden Ansprüche, **dadurch gekennzeichnet, dass** ein Konfigurationsverfahren eines Sensors ein Alarmsignal liefert, wenn zwei Sensoren dieselben Position zugewiesen wird; auf diese Weise können durch den Bediener verursachte Fehler bei der Positionierung der Sensoren vermieden werden.

14. Vorrichtung zur permanenten Überwachung der Vitalzeichen, bestehend aus:
a. Einem oder mehreren drahtlosen Sensoren (1.1, 1.2, 1.3), die auf der Körperoberfläche eines Patienten (1.6) für die Vitalzeichenüberwachung anwendbar sind, wobei jeder Sensor mit einem Sende-Empfangsgerät zum Senden und Empfangen von Basisband-Impulssignalen ausgestattet ist;
b. Mindestens einer Basiseinheit BU (1.4), die mit einem Sende-Empfangsgerät zum Senden und Empfangen von Basisband-Impulssignalen zu/von einem oder mehreren der Sensoren ausgestattet ist und mit mindestens einem Monitor (1.5) verbunden ist,
wobei mindestens ein Sensor (1.1, 1.2, 1.3) und eine Basiseinheit (1.4) Mittel umfassen, um durch die Nutzung von Ultrabreitband-Impulssignalen, die in zugewiesenen Zeitschlitzen übertragen werden, miteinander zu kommunizieren, mit einer Zeit- oder Codemultiplex-Kommunikation mit einer Basisband-Vielfachzugriffstechnik und **dadurch gekennzeichnet, dass** die Basiseinheit (1.4) zum Scannen aller durch einen neuen Sensor nutzbaren Codes und/oder Zeitschlitze konfiguriert ist, um alle belegten Codes oder Zeitschlitze von anderen aktiven Sensoren (2.1, 2.2, 2.3) zu erfassen, die letztendlich einer anderen Basiseinheit (2.4) gehören, und um dem neuen Sensor einen freien Code und/oder freien Zeitschlitz zuzuweisen, in dem er übertragen kann.

15. Vorrichtung nach Anspruch 14, **dadurch gekennzeichnet, dass** ein drahtloser Sensor einen rauscharmen Verstärker (4.3), einen Analog-Digital-Wandler (4.4), einen Mikrocontroller (4.7), einen DSP (4.5), einen Speicher (4.6), einen Puffer (4.8), einen Codierer/Decodierer (4.9), eine Batterie-Stromversorgung (4.10), ein Basisband-Sende-Empfangsgerät und eine Antenne beinhaltet, und die Basiseinheit eine Antenne (6.1), ein Basisband-Sende-Empfangsgerät (6.2), einen Codierer/Decodierer (6.3), einen Mikrocontroller (6.5) gekoppelt mit einem DSP (6.4) verbunden mit einer Benutzerschnittstelle, eine E/A-Schnittstelle (6.7) und einen Speicher (6.8), einen Demultiplexer (6.9), eine Reihe von Puffern, eine Reihe von Analog-Digital-Wandlern (jeweils pro Sensor), eine Batterie-Stromversorgung und eine Monitorschnittstelle beinhaltet.

## Revendications

1. Procédé pour la surveillance permanente des signes vitaux, comprenant l'opération consistant à :
a. positionner un ou plusieurs capteurs sans fil (1.1, 1.2, 1.3) sur la surface corporelle d'un patient (1.6) pour surveiller les signaux vitaux ; chaque capteur étant équipé d'un émetteur-récepteur (4.11) pour transmettre et recevoir les signaux d'impulsion en bande de base ;
b. mettre sous tension au moins une unité de base-UB (1.4) équipée d'un émetteur-récepteur pour transmettre et recevoir les signaux d'impulsion en bande de base de/à un ou plusieurs desdits capteurs et connectée à au moins un moniteur (1.5) ;
dans lequel au moins un capteur et une unité de base communiquent entre eux en utilisant des signaux d'impulsion à bande ultralarge transmis dans des créneaux affectés, avec une communication à multiplexage temporel ou répartition de code avec une technique d'accès multiple en bande de base et **caractérisé par le fait qu'**une phase de numérisation est en outre fournie et dans laquelle l'unité de base (1.4) scanne tous les codes et/ou les créneaux pouvant être utilisés par un nouveau capteur afin d'acquérir tous les codes ou les créneaux occupés d'autres capteurs actifs (2.1, 2.2, 2.3) éventuellement détenus par une autre unité de base (2.4), et d'assigner nouveau capteur précité un code et/ou des créneaux disponibles où il peut transmettre.

2. Procédé, selon la revendication 1, **caractérisé par le fait qu'**un capteur sans fil (1.1, 1.2, 1.3) et une unité de base (1.4) communiquent au moyen de signaux d'impulsion à large bande transmis dans des intervalles de temps distincts assignés à chaque dispositif avec une technique d'accès multiple par répartition dans le temps ou par division de code.

3. Procédé selon les revendications 1 ou 2, **caractérisé par le fait qu'**une unité de base (1.4) transmet un signal de référence temporelle global à tous les capteurs (1.1, 1.2, 1.3) afin de synchroniser la séquence temporelle de chaque capteur avec celle de l'unité de base.

4. Procédé selon la revendication 1, **caractérisé par le fait qu'**une unité de base (1.4) durant la phase de configuration recherche un signal de référence temporelle global généré par l'autre unité de base (2.4) et s'il existe, l'unité de base aligne sa séquence temporelle avec celui-ci.

5. Procédé selon une ou plusieurs des revendications précédentes **caractérisé par le fait qu'**une unité de base (1.4) envoie les paramètres de configuration ; ces paramètres sont envoyés par un canal de commande (160) à chaque capteur appartenant à l'unité de base ; le canal de commande est divisé en temps ou en code par le canal de données dans lequel la transmission des informations est basée sur une technique en bande de base.

6. Procédé selon une ou plusieurs des revendications précédentes **caractérisé par le fait que** l'unité de base (1.4) assigne à chaque capteur sans fil (1.1, 1.2, 1.3) un code spécifique et/ou un code spécifique de saut temporel selon lequel le capteur doit transmettre les impulsions du signal bio-potentiel provenant du corps du patient (1.6) ; l'unité de base assigne le code au cours d'une procédure de configuration spécifique du capteur qui commence avant le début des informations des signaux vitaux mesurés du patient.

7. Procédé selon une ou plusieurs des revendications précédentes **caractérisé par le fait qu'**une unité de base (1.4) a une interface à un moniteur standard (1.5) qui affiche les signaux bio-potentiels émanant des capteurs sans fil (1.1, 1.2, 1.3) exactement tel qu'ils proviennent des câbles classiques ; alternativement, une unité de base est connectée à un moniteur dédié ou est mise en oeuvre dans un moniteur dédié.

8. Procédé selon une ou plusieurs des revendications précédentes **caractérisé par le fait que** durant une phase de configuration précitée l'opérateur place les capteurs (1.1, 1.2, 1.3) un par un sur la surface du corps du patient (1.6) ; chaque capteur, une fois activé, transmet un signal de départ sur ledit canal de commande (160). L'unité de base (1.4) reçoit ledit signal de départ et demande à l'opérateur à travers un écran l'emplacement du capteur sur le corps du patient ; ensuite l'unité de base assigne le code au capteur ; le capteur cesse de transmettre sur le canal de commande et commence la transmission des données d'information sur le canal de données selon le code assigné ; l'unité de base stocke le code et l'emplacement de chaque capteur actif dans une mémoire (4.6).

9. Procédé selon une ou plusieurs des revendications précédentes **caractérisé par le fait qu'**une unité de base répète une procédure de configuration précitée jusqu'à ce qu'un code disponible et/ou créneau existe ou jusqu'à ce que l'opérateur saisisse une commande de configuration de fin.

10. Procédé selon une ou plusieurs des revendications précédentes **caractérisé par le fait que** chaque capteur peut être reconfiguré plusieurs fois par une unité de base ; chaque capteur peut être également reprogrammé avec une fonction ou un emplacement différent en fonction de l'examen spécifique souhaité.

11. Procédé selon une ou plusieurs des revendications précédentes **caractérisé par le fait qu'**une procédure de configuration peut être redémarrée à tout moment, de sorte que l'opérateur puisse substituer un capteur ou configurer un nouveau capteur sans interrompre ou altérer la transmission des autres capteurs, qui peuvent continuer à transmettre la surveillance des signes vitaux.

12. Procédé selon une ou plusieurs des revendications précédentes **caractérisé par le fait que** dans un capteur sans fil précité les données sont envoyées au modulateur qui crée les trains d'impulsion, selon le code assigné au capteur par l'unité de base au cours de la phase de configuration. Les données sont modulées par plusieurs impulsions radio en bande de base tel que spécifié par le code du capteur spécifique. Le signal modulé est converti en analogique, amplifié et envoyé à l'antenne (5.7) pour la transmission.

13. Procédé selon une ou plusieurs des revendications précédentes **caractérisé par le fait qu'**une procédure de configuration précitée d'un capteur fournit un signal d'alarme lorsque le même emplacement est assigné à deux capteurs ; de cette façon les erreurs dans le positionnement des capteurs par l'opérateur peuvent être évitées.

14. Dispositif pour la surveillance permanente des signes vitaux, composé :
a. d'un ou de plusieurs capteurs sans fil (1.1, 1.2, 1.3) pouvant s'appliquer sur la surface corporelle d'un patient (1.6) pour la surveillance des signes vitaux ; chaque capteur étant équipé d'un émetteur-récepteur pour transmettre et recevoir les signaux d'impulsion en bande de base ;
b. au moins une unité de base-UB (1.4) équipée d'un émetteur -récepteur pour transmettre et recevoir les signaux d'impulsion en bande de base de/à un ou plusieurs desdits capteurs précités et connectée à au moins un moniteur (1.5),
dans lequel au moins un capteur (1.1, 1.2, 1.3) et une unité de base (1.4) comprennent des moyens pour communiquer entre eux en utilisant des signaux d'impulsion à bande ultralarge transmis dans des créneaux affectés, avec une communication à multiplexage temporel ou par répartition de code avec une technique d'accès multiple en bande de base et **caractérisé par le fait que** l'unité de base (1.4) est configurée pour scanner tous les codes et/ou les créneaux utilisables par un nouveau capteur afin d'acquérir tous les codes ou les créneaux occupés d'autres capteurs actifs (2.1, 2.2, 2.3) éventuellement détenus par l'autre unité de base (2.4), et d'assigner audit nouveau capteur un code et/ou des créneaux disponibles où il peut transmettre.

15. Dispositif, selon la revendication 14, **caractérisé par le fait qu'**un capteur sans fil comprend un amplificateur à faible bruit (4.3), un convertisseur analogique/numérique (4.4), un microcontrôleur (4.7), un DSP (4.5), une mémoire (4.6), un tampon (4.8), un codeur/décodeur (4.9), une alimentation par batterie (4.10), un émetteur-récepteur en bande de base (6.2) et une antenne et l'unité de base comprend une antenne (6.1), un codeur/décodeur (6.3), un microcontrôleur (6.5) couplé avec un DSP (6.4) connecté à une interface utilisateur, une interface E/S (6.7) et une mémoire (6.8), un démultiplexeur (6.9), un banc de tampons, un banc de convertisseurs analogiques/numériques (pour chaque capteur), une alimentation par batterie et une interface moniteur.
